# FASCICULE DE BREVET EUROPEEN

(11) **EP 2 155 648 B1**
(45) Date de publication et mention de la délivrance du brevet: **12.09.2012**
(21) Numéro de dépôt: 08759929.6
(22) Date de dépôt: 22.05.2008
(51) Int. Cl.: C07C 51/41, C07C 55/02, C07C 55/14, C07C 211/09, C07C 211/12, C08G 69/28

(54) **PROCEDE DE FABRICATION D'UNE SOLUTION DE SELS DE DIACIDES/DIAMINES**
VERFAHREN ZUR HERSTELLUNG EINER LÖSUNG AUS DIACID-/DIAMIN-SALZEN
PROCESS FOR PRODUCING A SOLUTION OF DIACID/DIAMINE SALTS

(30) Priorité: 04.06.2007 FR 0703933
(43) Date de publication de la demande: 24.02.2010
(73) Titulaire: Rhodia Opérations, 93306 Aubervilliers (FR)
(72) Inventeur: THIERRY, Jean-François, 69340 Francheville (FR)
(74) Mandataire: Chatelan, Florence Anne
(86) Numéro de dépôt international: PCT/EP2008/056328
(87) Numéro de publication internationale: WO 2008/148647

(56) Documents cités:
- US-A- 4 213 884
- US-A- 4 233 234
- US-A- 4 442 260
- US-A- 5 801 278

## Description

La présente invention concerne un procédé de fabrication d'une solution d'un sel de diamine et de diacide pour la fabrication de polyamide.

Plus particulièrement, l'invention concerne un procédé pour la fabrication d'une solution concentrée de sel adipate d'hexaméthylène diammonium, appelé également sel Nylon, utilisé comme matière première pour la fabrication de polyamide, plus précisément de PA66.

Pour obtenir des polyamides à monomères diacides et diamines de poids moléculaire élevé, on utilise généralement une solution aqueuse d'un sel formé par réaction entre une molécule de diamine et une molécule de diacide. Cette solution est chauffée pour évaporer l'eau dans un premier temps puis pour engager la polymérisation par polycondensation, pour obtenir des chaînes macromoléculaires comprenant des fonctions amides.

La solution de sel contient généralement des quantités stoechiométriques de diacides et diamines. La concentration pondérale en sel Nylon est généralement comprise entre 50% et 65%. Cette solution est généralement stockée avant d'être transportée le cas échéant, puis alimentée dans les installations de polymérisation.

La concentration maximale en sel Nylon admissible pour éviter les problèmes de précipitation est de l'ordre de 70% en poids à pression atmosphérique. Au-delà de cette concentration, il est nécessaire de chauffer la solution à des températures comprises entre 110 et 160°C, sous une pression supérieure à la pression atmosphérique, pour éviter toute précipitation. Ces domaines de température et de pression sont difficilement compatibles avec le stockage et le transport.

Plusieurs procédés de fabrication de solution de sel Nylon ont été proposés. Ces procédés consistent généralement à ajouter l'acide adipique dans l'hexaméthylène diamine et de l'eau en évacuant la chaleur produite par la réaction de neutralisation.

Ainsi, le brevet US 4 233 234 décrit un procédé de fabrication d'un adipate d'hexaméthylène diammonium comprenant un réacteur mélangeur et une circulation de la solution dans une zone de mélange, puis dans un réfrigérant pour évacuer les calories générées par la réaction entre le diacide et la diamine.

Le brevet US 4 442 260 décrit un procédé consistant à produire une solution aqueuse contenant de 31 à 40% d'eau, 73,5 à 77,5% d'acide adipique et 22,5 à 26,5% d'hexaméthylène diamine, puis à évaporer une grande partie de l'eau pour obtenir une concentration pondérale en sel non stoechiométrique de 89 à 96%, et additionner de l'hexaméthylène diamine pour obtenir un rapport stoechiométrique diacide/diamine égal à 1.

Ces différents procédés de fabrication nécessitent d'une part l'apport de calories notamment pour dissoudre l'acide adipique et d'autre part, l'évacuation des calories générées par la réaction entre l'amine et l'acide, et également d'évaporer de l'eau.

Un des buts de la présente invention est de proposer un procédé de préparation d'une solution concentrée en Sel Nylon ou sel de diacide et de diamine, utilisant le minimum d'échange d'énergie avec l'extérieur, c'est à dire minimisant l'apport et l'évacuation de calories.

A cet effet, l'invention propose un procédé de fabrication de solution aqueuse de sels de diamines et diacides obtenus par mélange d'un diacide et d'une diamine, à une concentration pondérale en sel comprise entre 50 et 80%, caractérisé en ce qu'il consiste à :
- Produire dans un premier réacteur une solution aqueuse de diamine et diacide avec un rapport molaire diacide/diamine compris ente 1,5 et 5 et une concentration dans l'eau des espèces dissoutes comprise entre 40 et 75% en poids, préférentiellement 45 et 65%, par alimentation dans ledit réacteur contenant soit au moins 5% en volume de solution aqueuse de diamine et diacide avec un rapport molaire compris entre 1,5 et 5, soit de l'eau représentant au moins 10% de la quantité totale d'eau à alimenter dans ledit réacteur, d'un flux comprenant du diacide, d'un flux comprenant de la diamine et éventuellement d'un flux d'eau à la température T₁, les débits du flux d'alimentation comprenant du diacide et du flux d'alimentation comprenant de la diamine étant contrôlés pour avoir constamment une température de la solution dans le réacteur inférieure à la température d'ébullition sous la pression opératoire de celui-ci et un rapport molaire diacide/diamine supérieur à 1,1, la quantité d'acide alimentée correspondant à au moins 90% en poids de la masse totale de l'acide nécessaire pour produire la quantité désirée de solution aqueuse de sel, la quantité d'eau alimentée représentant au moins 75% en poids de la masse totale d'eau nécessaire pour produire la quantité désirée de la solution aqueuse de sel,
- Transférer la solution aqueuse obtenue dans le premier réacteur dans un deuxième réacteur équipé d'un condenseur,
- Alimenter dans le deuxième réacteur un flux comprenant de la diamine pour obtenir un rapport molaire diacide/diamine compris entre 0,9 et 1,1, de préférence entre 1,0 et 1,05, la solution étant portée à une température au plus égale à la température d'ébullition de la solution à la pression opératoire par au moins le dégagement de chaleur de la réaction entre la diamine et le diacide, et éventuellement alimenter les quantités d'eau et/ou de diacide complémentaire pour obtenir la solution de sel à la concentration et au rapport diacide/diamine désirés.

Par espèces dissoutes, il faut comprendre l'ensemble des espèces diacides et diamines présentes dans le milieu sous forme libre ou de sel ou autre.

Par température d'ébullition, il faut comprendre la température d'ébullition de la solution contenue dans un réacteur à la pression de travail ou opératoire du procédé.

Comme diamines convenables pour l'invention on peut citer l'hexaméthylène diamine (HMD) comme monomère préféré et le plus utilisé, et également l'heptaméthylène diamine, la tétraméthylène diamine, l'octaméthylène diamine, la nonaméthylène diamine, la décaméthylène diamine, la méthyl-2 pentaméthylène diamine, l'undécaméthylène diamine, la dodécaméthylène diamine, la xylylène diamine, l'isophorone diamine. Il est possible d'utiliser un mélange de plusieurs monomères diamines.

Dans le procédé de l'invention, la diamine est alimentée sous forme pure ou, de préférence, sous forme de solution aqueuse concentrée. Pour l'HMD, une solution comprenant au moins 50% en poids de diamine, de préférence au moins 85% et encore plus avantageusement 90% en poids environ est préférentiellement utilisée. Toutefois, le flux comprenant la diamine peut contenir d'autres composés sans pour cela sortir du cadre de l'invention.

Comme diacides convenables pour l'invention, on peut citer les acides subérique, sébacique, dodécanedioïque, isophtalique, téréphtalique, azélaïque, pimélique, naphtalène dicarboxylique, par exemple. Il est possible d'utiliser un mélange de plusieurs monomères diacides. L'acide adipique est le monomère préféré et le plus utilisé. Il est mis en oeuvre sous forme de poudre. Toutefois, il peut également être alimenté dans le réacteur sous forme de solution aqueuse ou de suspension.

Comme pour le flux comprenant la diamine, le flux comprenant le diacide peut contenir d'autres composés, solvants, sans pour cela sortir du cadre de l'invention.

En outre, les flux de produits alimentés dans le premier réacteur peuvent être, de préférence, distincts. Toutefois, la diamine peut être ajoutée avec l'eau et une partie du diacide. De même, le diacide est alimenté, de préférence, sous forme de poudre. Toutefois, il peut être alimenté sous forme de solution ou dispersion aqueuse ou sous forme dissoute, par exemple, dans une solution aqueuse de sel diamine/diacide, sans pour cela sortir du cadre de l'invention.

Le procédé de l'invention est réalisé avantageusement en maintenant les différents réacteurs, plus particulièrement les deuxième et troisième réacteurs décrits ci-après, sous atmosphère exempte d'oxygène, comme par exemple, sous une atmosphère constituée par de l'azote, des gaz rares, de la vapeur d'eau ou un mélange de ceux-ci.

Dans un mode de réalisation préféré, l'atmosphère exempte d'oxygène est obtenue soit par alimentation en continu d'un flux d'azote, soit par maintien d'une pression d'azote dans les différents réacteurs et par génération de vapeur d'eau par l'ébullition de la solution.

Dans ce dernier cas, il est avantageux que l'échappement ou évacuation de l'azote, soit réalisé à travers un condenseur monté sur le réacteur. Ainsi, l'eau entraînée avec l'azote est condensée et recyclée dans le réacteur.

Ce mode de réalisation permet également l'évacuation de l'oxygène présent, par exemple sous forme dissoute, dans la solution et donc évite une oxydation des monomères, notamment de la diamine. L'oxygène peut être apporté, notamment, par le monomère diacide.

Dans un autre mode de réalisation, l'oxygène dissous est évacué par entraînement par la vapeur d'eau générée par l'ébullition de la solution dans le second réacteur, quand la température de la solution est égale à cette température d'ébullition.

Le procédé de l'invention peut être réalisé selon un mode discontinu ou un mode continu. Ces deux modes de réalisation sont décrits en détail ci-dessous.

Le procédé de l'invention peut être mis en oeuvre dans tout type de réacteur. Plus particulièrement, les réacteurs comprennent une agitation mécanique et peuvent être équipés de moyens permettant de les maintenir en température, notamment pendant les périodes d'arrêt ou de changement de campagne de fabrication.

Dans le mode de réalisation en discontinu, le procédé de l'invention est mis en oeuvre, de manière préférée, dans une installation comprenant plusieurs réacteurs montés en série, chaque réacteur correspondant à la réalisation d'une étape du procédé. Toutefois, sans sortir du cadre de l'invention, les différentes étapes du procédé peuvent être réalisées successivement dans le même réacteur. De même, l'installation peut comprendre plusieurs réacteurs montés en parallèle pour la réalisation d'une étape du procédé.

La solution concentrée de sel obtenue selon le procédé de l'invention peut être alimentée directement et en continu dans une installation de polymérisation, ou peut être stockée avant transfert et utilisation.

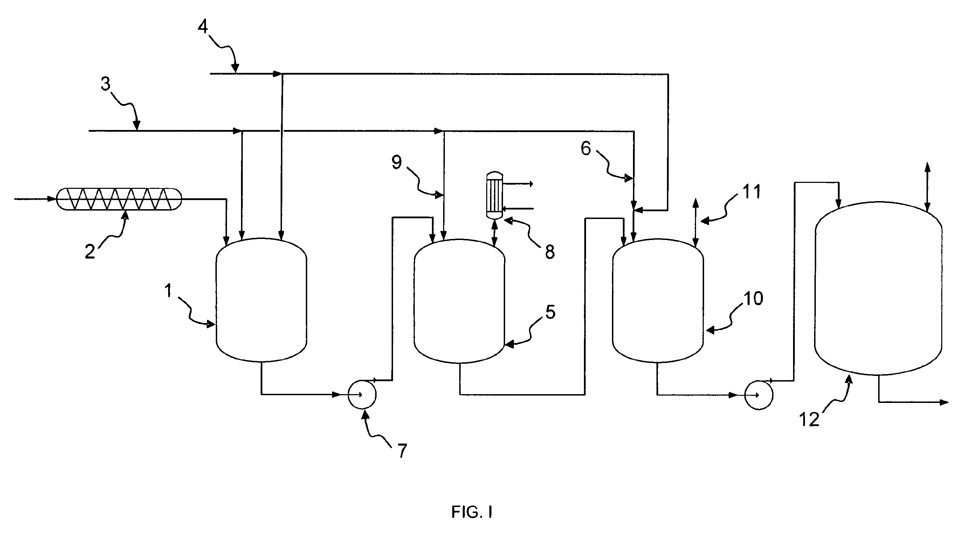

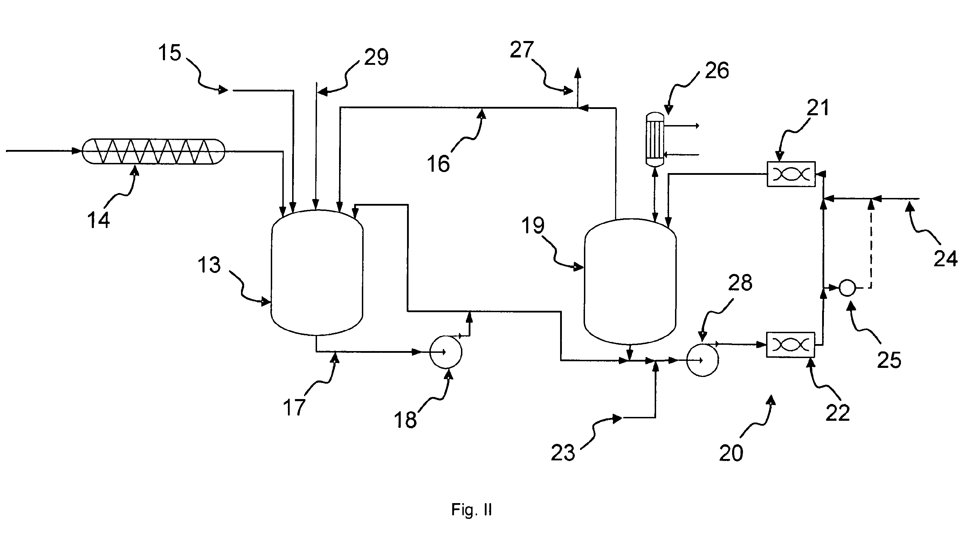

Une description détaillée de deux modes de réalisation du procédé de l'invention est réalisée ci-dessous en référence aux figures 1 et 2 annexées dans lesquelles :
- la figure 1 représente un schéma synoptique d'une installation permettant de mettre en oeuvre le procédé selon un mode de réalisation discontinu, et
- la figure 2 représente un schéma synoptique d'une installation permettant de mettre en oeuvre le procédé selon un mode de réalisation continu.

L'invention est également illustrée par les exemples de fabrication de solutions concentrées en sel Nylon obtenues selon le mode de réalisation en discontinu du procédé.

Dans la description ci-dessous on utilisera les termes acide adipique (AA) et hexaméthylène diamine (HMD) pour désigner le diacide et la diamine. Toutefois, ce procédé s'applique également à d'autres diacides et d'autres diamines indiqués précédemment.

En référence à la figure 1, on décrit un premier mode de réalisation du procédé de l'invention fonctionnant selon le mode discontinu. L'installation comprend un premier réacteur 1 agité, dans lequel sont ajoutés de l'acide adipique 2, généralement sous forme d'une poudre, et un flux 3 liquide d'hexaméthylène diamine. De l'eau 4 est également introduite dans ce réacteur.

L'hexaméthylène diamine est avantageusement une solution aqueuse concentrée comprenant 90% en poids d'HMD.

Les différents produits sont ajoutés dans le réacteur 1 qui contient une faible quantité de solution d'acide adipique et d'hexaméthylène diamine dans de l'eau, riche en acide adipique, et appelée pied de solution. Cette solution aqueuse est avantageusement une faible partie de la solution préparée dans une opération antérieure et a, avantageusement, comme composition, sensiblement la composition finale de la solution qui sera préparée dans ce réacteur 1, à savoir un rapport molaire diacide/diamine égal à environ 2,4 et une concentration pondérale en espèces dissoutes d'environ 57%.

La quantité de solution présente dans le réacteur en début d'étape est égale à au moins environ 5%, de préférence entre 5% et 40%, préférentiellement entre 10% et 35% de la quantité totale de solution produite dans le réacteur 1, en fin d'étape.

Avantageusement, selon une caractéristique de l'invention aucun échange de chaleur n'est mis en oeuvre entre le réacteur et l'environnement, ou l'extérieur, c'est-à-dire que ce réacteur fonctionne en mode quasi adiabatique.

La température dans le réacteur 1 s'élève légèrement à cause de la réaction de neutralisation entre l'HMD et l'acide adipique. Toutefois, la température de la solution dans le réacteur, pendant toute l'opération et en fin d'étape, sera toujours à une température basse avantageusement inférieure à 100°C, de préférence inférieure à 75°C, et plus généralement inférieure à la température d'ébullition de la solution sous la pression opératoire. Ce niveau de température bas est avantageux pour limiter les phénomènes d'oxydation de l'HMD par l'oxygène présent dans le milieu. Cet oxygène peut être notamment apporté par la poudre d'acide adipique.

Quand les quantités d'eau, d'acide adipique et HMD nécessaires pour obtenir une solution aqueuse contenant de l'acide adipique, du sel de diacide et diamine, avec un rapport molaire global diacide/diamine égal à 2,4 et une concentration pondérale en espèces dissoutes de 57% sont alimentées dans le réacteur. Quand le volume de milieu réactionnel dans le réacteur représente avantageusement au moins 80% du volume utile du réacteur 1, la solution est alimentée dans un second réacteur 5 appelé réacteur de neutralisation, par une pompe 7. Ce réacteur 5 est équipé d'un condenseur 8 et avantageusement d'une boucle externe de circulation de la solution et/ou d'un agitateur (non représentés).

Dans ce deuxième réacteur 5, de l'hexaméthylène diamine 9 est alimentée pour obtenir un rapport molaire AA/HMD voisin de 1,01. Comme pour le premier réacteur 1, aucun échange de chaleur significatif n'est avantageusement réalisé avec l'extérieur. Ainsi la chaleur de la réaction de neutralisation de l'amine par l'acide provoque une augmentation de la température dans le réacteur 5 pour atteindre au maximum la température d'ébullition du mélange à la pression opératoire. L'eau qui s'évapore est condensée dans le condenseur 8 pour obtenir un reflux total de l'eau. Cette caractéristique de température pour obtenir une ébullition est avantageuse car elle permet d'éliminer, par entraînement à la vapeur d'eau, l'oxygène présent dans le milieu notamment sous forme dissoute. L'échange de chaleur réalisé dans ce condenseur est très faible et ne représente qu'une très petite partie de la chaleur dégagée par la réaction de neutralisation.

De l'eau peut être également ajoutée pour ajuster la concentration en sel d'adipate d'hexaméthylène ammonium à une concentration pondérale supérieure à 50%, de préférence comprise entre 60 et 75% en poids. L'eau peut être avantageusement mélangée au flux d'hexaméthylène diamine.

Dans le mode illustré qui est le mode préféré de l'invention, la solution obtenue dans le deuxième réacteur 5 est alimentée dans un troisième réacteur 10 muni d'une agitation (non représentée) et avantageusement d'un condenseur 11.

Ce troisième réacteur 10, appelé également réacteur d'ajustement, est analogue dans son principe au second réacteur et comprend une addition 6 d'HMD et d'eau pour ajuster le rapport AA/HMD à une valeur comprise entre 0,995 et 1,005, et ajuster si nécessaire, la concentration en sel à la valeur désirée.

La solution ainsi obtenue peut être utilisée directement dans une installation de polymérisation ou être stockée dans un réservoir de stockage 12 ou dans des conteneurs adaptés pour le transport.

Selon un mode de réalisation préféré, les réacteurs de l'installation sont maintenus sous atmosphère exempte d'oxygène par alimentation, par exemple, d'azote dans le réacteur vide, et maintien de cette atmosphère d'azote lors des remplissages et vidanges des réacteurs. Les alimentations en azote de chaque réacteur ne sont pas représentées sur la figure annexée.

Avantageusement, les réacteurs sont munis d'une isolation thermique pour limiter les échanges de chaleur avec le milieu extérieur et ainsi limiter les pertes de chaleur.

Dans ce mode de réalisation, l'oxygène dissous sera évacué par entraînement par l'azote qui s'échappe du réacteur au cours du remplissage de celui-ci. Cette évacuation de l'azote est effectuée, de préférence, à travers un condenseur pour ainsi condenser la vapeur d'eau entraînée par l'azote.

En se référant à la figure 2, un second mode de réalisation du procédé de l'invention est décrit. Ce second mode de réalisation concerne un procédé fonctionnant selon un mode continu. Comme dans le premier mode de réalisation, le procédé comprend une première étape de dissolution de l'acide adipique mise en oeuvre dans le réacteur 13. L'acide adipique est alimenté par un système à vis sans fin 14, simultanément à de l'eau 15 et à un flux 16 de solution de sel de Nylon concentrée pour obtenir dans le réacteur 13 une solution contenant un rapport molaire diacide/diamine compris entre 1,5 et 5, de préférence voisin de 2,4, et une concentration pondérale en espèces dissoutes comprise entre 40 et 75%, par exemple égale à 57%. Dans un autre mode de réalisation, la diamine est alimentée dans le réacteur 13 par un flux indépendant du flux de solution concentrée en sel, l'alimentation totale en diamine dans le réacteur 13 pouvant être obtenue soit par la seule alimentation de diamine soit par le flux de solution concentrée en sel ou encore par l'addition de ces deux flux.

Pour obtenir une homogénéisation de la solution dans le réacteur 13, une boucle de circulation externe 17 comprenant une pompe 18 est illustrée. Une partie de la solution circulant dans la boucle alimente un réacteur 19 également équipé d'une boucle externe 20 de neutralisation comprenant une pompe 28 et deux mélangeurs statiques 21 et 22. En amont de chaque mélangeur est située une alimentation 23 en HMD et une alimentation 24 en monomères pour ajuster le rapport molaire diacide/diamine à une valeur comprise entre 0,99 et 1,01.

Ce rapport molaire est avantageusement contrôlé et ajusté par la mesure 25 du pH de la solution et l'addition de diamine et/ou diacide complémentaire en aval de la mesure de pH. Comme dans le premier mode de réalisation, la chaleur dégagée par la neutralisation permet une augmentation de la température de la solution jusqu'à atteindre, au maximum, la température d'ébullition de la solution à la pression opératoire.

Pour condenser l'eau ainsi évaporée, un condenseur 26 est prévu sur le réacteur 19.

Dans le mode de réalisation illustré, une partie de la solution produite dans le réacteur 19 est envoyée dans le premier réacteur 13 par le conduit 16, l'autre partie 27 est dirigée vers des stockeurs non représentés.

Il est possible de ne pas recycler la solution de sel concentrée dans le premier réacteur. Dans ce cas, la diamine est introduite dans le premier réacteur par une alimentation distincte 29 sous forme pure ou solution aqueuse. Les deux alimentations 16 et 29 de diamine dans le premier réacteur peuvent être présentes simultanément.

Les exemples ci-dessous illustrent plus clairement le procédé de l'invention et les caractéristiques et avantages de celui-ci.

Exemple 1 - Production d'une solution aqueuse de Sel Nylon à 62% en poids, selon le procédé discontinu.

Etape 1 - Dissolution de l'Acide Adipique

Une solution aqueuse d'acide adipique et d'hexaméthylène diamine est préparée en ajoutant de l'acide adipique en poudre (35,0 kg) et de l'hexaméthylène diamine (12,9 kg d'une solution aqueuse à 90% en poids à une température de 45°C) dans le réacteur 1, isolé thermiquement, contenant une solution aqueuse obtenue par addition d'eau (34,4 kg; à une température de 40°C) dans un pied de 14 kg de solution aqueuse d'acide adipique et d'hexaméthylène diamine présentant un rapport molaire AA/HMD = 2,4, à une température de 63°C et une concentration pondérale en espèces dissoutes de 56,6%.

Ce pied de solution est une faible partie de la solution obtenue dans le réacteur 1 dans l'opération de fabrication précédente. L'acide adipique et l'HMD sont ajoutés simultanément en veillant à ce que le mélange soit toujours en excès acide (rapport molaire AA/HMD supérieur à 1,1). L'homogénéité de la solution est assurée par agitation mécanique. En fin de première étape, les espèces dissoutes (56,6% en poids) consistent en 75,1% en poids d'acide adipique et 24,9% en poids d'hexaméthylène diamine. La température finale de la solution est de 63°C.

Etape 2 - Neutralisation

82,3 kg (soit environ 85,5%) de la solution obtenue dans l'étape 1 sont transférés dans le réacteur 5 de la figure 1, isolé thermiquement et équipé d'un condenseur 8. Une partie de la solution obtenue est conservée comme pied de solution dans le réacteur 1 pour une opération ultérieure.

17,5 kg d'une solution aqueuse d'hexaméthylène diamine à 90% en poids d'HMD et à une température de 45°C, sont additionnés dans le réacteur 5 pour obtenir une solution aqueuse de sel Nylon dont le rapport diacide/diamine est proche de la stoechiométrie (Rapport Molaire AA/HMD = 1,017).

L'énergie ou chaleur dégagée par la réaction de neutralisation provoque l'élévation de la température du milieu jusqu'à la température d'ébullition, à savoir 108°C dans l'exemple décrit. Les vapeurs produites sont condensées dans le condenseur 8 et forment un reflux total dans le réacteur 5. L'énergie évacuée par la condensation des vapeurs correspond à l'énergie de neutralisation excédentaire. Ainsi, le procédé permet de maintenir le système à la température de 108°C (début d'ébullition à pression atmosphérique), sans nécessiter l'utilisation de moyens pour évacuer les calories générées par la réaction de neutralisation.

Etape 3 - Finition

La concentration et le pH de la solution sont ensuite ajustés par addition de 0,9 kg d'eau à une température de 40°C et de 0,43 kg d'une solution aqueuse d'HMD à 90% en poids et à une température de 45°C, après transfert de la solution dans un troisième réacteur. A la fin de cette étape, la solution est une solution aqueuse contenant 62,0% en poids de sel Nylon avec un rapport molaire acide adipique/HMD égal à 1,003 et un pH égal à 7,21. Le pH est mesuré à 20°C sur un échantillon de la solution dilué avec de l'eau pour obtenir une concentration en espèces dissoutes égale à 100 g/L.

La solution obtenue est ensuite stockée dans un réservoir 12 illustré à la figure 1.

Exemple 2 - Production de Sel Nylon à 68% selon le procédé discontinu.

Etape 1 - Dissolution de l'Acide Adipique

Une solution aqueuse d'acide adipique et d'hexaméthylène diamine est préparée en ajoutant 38,0 kg d'acide adipique en poudre et 14,0 kg d'une solution aqueuse d'hexaméthylène diamine à 90% en poids d'HMD et à une température de 45°C, dans le réacteur 1 contenant une solution aqueuse obtenue par addition de 27,7 kg d'eau à une température de 40°C, dans un pied de 14 kg de solution aqueuse d'acide adipique et d'hexaméthylène diamine présentant un rapport molaire AA/HMD = 2,4, à une température de 68°C et une concentration pondérale en espèces dissoutes de 63,5%.

Ce pied de solution est une faible partie de la solution obtenue dans le réacteur 1 dans l'opération de fabrication précédente. L'acide adipique et l'HMD sont ajoutés simultanément en veillant à ce que le mélange soit toujours en excès acide (Rapport Molaire AA/HMD supérieur à 1,1). L'homogénéité de la solution est assurée par agitation mécanique. En fin de première étape, les espèces dissoutes (63,5% en poids) consistent en 75,1 % en poids d'acide adipique et 24,9% en poids d'hexaméthylène diamine. La température finale de la solution est de 68°C.

Etape 2 - Neutralisation

79,7 kg (soit environ 85%) de la solution obtenue dans l'étape 1 sont transférés dans le réacteur 5 de la figure 1 équipé d'un condenseur 8. Une partie de la solution obtenue est conservée comme pied de solution dans le réacteur 1 pour une opération ultérieure.

19,0 kg d'une solution aqueuse d'hexaméthylène diamine à 90% en poids d'HMD et à une température de 45°C, sont additionnés dans le réacteur 5 pour obtenir une solution aqueuse de sel Nylon dont le rapport diacide/diamine est proche de la stoechiométrie (Rapport Molaire AA/HMD = 1,017).

L'énergie ou chaleur dégagée par la réaction de neutralisation provoque l'élévation de la température du milieu jusqu'à la température d'ébullition, à savoir 110°C, dans l'exemple décrit. Les vapeurs produites sont condensées dans le condenseur 8 et forment un reflux total dans le réacteur 5. L'énergie évacuée par la condensation des vapeurs correspond à l'énergie de neutralisation excédentaire. Ainsi, le procédé permet de maintenir le système à la température de 110°C (début d'ébullition à pression atmosphérique).

Etape 3 - Finition

La concentration et le pH de la solution sont ensuite ajustés par addition de 1,0 kg d'eau à une température de 40°C et 0,47 kg d'une solution aqueuse d'HMD à 90% en poids d'HMD et à une température de 45°C, après transfert de la solution dans un troisième réacteur 10 muni d'un condenseur 11. A la fin de cette étape, la solution est une solution aqueuse contenant 68,0% en poids de sel Nylon avec un Rapport Molaire AA/ HMD égal à 1,003.

## Revendications

1. Procédé de fabrication de solution aqueuse de sel de diamines et diacides obtenus par mélange d'un diacide et d'une diamine, à une concentration pondérale en sel comprise entre 50 et 80%, **caractérisé en ce qu'**il consiste à :
- Produire dans un premier réacteur une solution aqueuse de diamine et diacide avec un rapport molaire diacide/diamine compris ente 1,5 et 5 et une concentration dans l'eau des espèces dissoutes comprise entre 40 et 75% en poids, par alimentation dans ledit réacteur contenant soit au moins 5% en volume de solution aqueuse de diamine et diacide avec un rapport molaire compris entre 1,5 et 5, soit de l'eau représentant au moins 10% de la quantité totale d'eau à alimenter dans ledit réacteur, d'un flux comprenant du diacide, d'un flux comprenant de la diamine et éventuellement d'un flux d'eau à la température T₁, les débits des flux d'alimentation comprenant du diacide et du flux d'alimentation comprenant de la diamine étant contrôlés pour avoir constamment une température de la solution dans le réacteur inférieure à la température d'ébullition sous la pression opératoire de celui-ci et un rapport molaire diacide/diamine supérieur à 1,1, la quantité d'acide alimentée correspondant à au moins 90% en poids de la masse totale de l'acide nécessaire pour produire la quantité désirée de solution aqueuse de sel, la quantité d'eau alimentée représentant au moins 75% en poids de la masse totale d'eau nécessaire pour produire la quantité désirée de la solution aqueuse de sel,
- Transférer la solution aqueuse obtenue dans le premier réacteur dans un second réacteur équipé d'un condenseur,
- Alimenter dans le second réacteur un flux comprenant de la diamine pour obtenir un rapport molaire diacide/diamine compris entre 0,9 et 1,1, la solution étant portée à une température au plus égale à la température d'ébullition de la solution à la pression opératoire par au moins le dégagement de chaleur de la réaction entre la diamine et le diacide, et éventuellement alimenter les quantités d'eau et/ou de diacide complémentaire pour obtenir la solution de sel à la concentration et au rapport diacide/diamine désirés.

2. Procédé selon la revendication 1, **caractérisé en ce que** la concentration en espèces dissoutes dans le premier réacteur est comprise entre 45 et 65% en poids.

3. Procédé selon la revendication 1 ou 2, **caractérisé en ce que** le rapport molaire diacide/diamine dans le deuxième réacteur est compris entre 1,00 et 1,05.

4. Procédé selon l'une des revendications 1 à 3, **caractérisé en ce que** la solution obtenue dans le deuxième réacteur est transférée dans un troisième réacteur maintenu de préférence en l'absence d'oxygène, muni d'un condenseur, dans lequel sont ajoutés un flux comprenant de la diamine et/ou un flux comprenant du diacide, et éventuellement un flux d'eau pour ajuster le rapport molaire diacide/diamine entre 0,99 et 1,01 et ajuster la concentration pondérale en sel.

5. Procédé selon l'une des revendications 1 à 4, **caractérisé en ce que** au moins les deuxième et troisième réacteurs sont maintenus sous atmosphère exempte d'oxygène.

6. Procédé selon l'une des revendications précédentes, **caractérisé en ce que** au moins les deuxième et troisième réacteurs sont sous une atmosphère exempte d'oxygène constituée par de l'azote, un gaz rare, de la vapeur d'eau ou un mélange de ceux-ci.

7. Procédé selon l'une des revendications précédentes, **caractérisé en ce qu'**il fonctionne en discontinu.

8. Procédé selon la revendication 7, **caractérisé en ce que** la solution aqueuse formant le pied de solution aqueuse représente au moins 5% en volume, de la quantité désirée de solution produite dans le réacteur en fin de première étape.

9. Procédé selon la revendication 8, **caractérisé en ce que** la solution aqueuse formant le pied de solution aqueuse représente entre 5% et 40% en poids, de préférence entre 10% et 35% en poids, de la quantité désirée de solution produite dans le réacteur en fin de première étape.

10. Procédé selon l'une des revendications précédentes, **caractérisé en ce que** la température dans le second réacteur est égale, au maximum, à la température d'ébullition de la solution à la pression opératoire, le condenseur générant un reflux d'eau condensée.

11. Procédé selon l'une des revendications précédentes, **caractérisé en ce que** la température dans le troisième réacteur est égale, au maximum, à la température d'ébullition de la solution à la pression opératoire, le condenseur générant un reflux d'eau condensée.

12. Procédé selon l'une des revendications précédentes, **caractérisé en ce que** le flux comprenant de la diamine alimenté dans le premier réacteur est une solution aqueuse de diamine.

13. Procédé selon la revendication 12, **caractérisé en ce que** la concentration en diamine dans la précitée solution aqueuse est au moins de 50% et, de préférence, au moins de 85% en poids.

14. Procédé selon l'une des revendications 1 ou 6, **caractérisé en ce que** le procédé fonctionne en continu.

15. Procédé selon la revendication 14, **caractérisé en ce que** le flux comprenant de la diamine alimenté dans le premier réacteur est constitué au moins partiellement par de la solution concentrée de sel produite par le procédé.

16. Procédé selon la revendication 14 ou 15, **caractérisé en ce que** le second réacteur comprend une boucle externe de circulation comprenant au moins un mélangeur statique.

17. Procédé selon la revendication 16, **caractérisé en ce que** l'alimentation du flux comprenant de la diamine dans le second réacteur est située en amont du mélangeur statique.

18. Procédé selon la revendication 17, **caractérisé en ce que** l'alimentation du flux de solution de sel provenant du premier réacteur est située en amont de l'alimentation en diamine.

19. Procédé selon l'une des revendications 16 à 18, **caractérisé en ce qu'**une mesure de pH de la solution est présente en aval du mélangeur statique, et **en ce que** la stoechiométrie est ajustée par alimentation d'un flux de diamine et/ou de diacide, en aval du point de mesure du pH et en fonction de cette mesure.

20. Procédé selon l'une des revendications précédentes, **caractérisé en ce que** la diamine est l'hexaméthylène diamine et le diacide est l'acide adipique.

## Claims

1. Process for the manufacture of an aqueous solution of salts of diamines and diacids, obtained by mixing a diacid and a diamine, at a salt concentration by weight of between 50% and 80%, **characterized in that** it consists in:
- producing, in a first reactor, an aqueous solution of diamine and diacid with a diacid/diamine molar ratio of between 1.5 and 5 and a concentration of the dissolved species in water of between 40% and 75% by weight by feeding into said reactor containing either at least 5% by volume of aqueous solution of diamine and diacid with a molar ratio of between 1.5 and 5, or water representing at least 10% of the total amount of water to be fed into said reactor, a stream comprising diacid, a stream comprising diamine and, optionally, a stream of water at the temperature T₁, the flow rates of the feed stream comprising diacid and of the feed stream comprising diamine being controlled so that the temperature of the solution in the reactor is constantly below the boiling point at the operating pressure of said reactor and so that the diacid/diamine molar ratio is constantly greater than 1.1, the amount of acid fed corresponding to at least 90% by weight of the total mass of the acid required to produce the desired amount of aqueous salt solution, the amount of water fed representing at least 75% by weight of the total mass of water required to produce the desired amount of the aqueous salt solution;
- transferring the aqueous solution obtained in the first reactor into a second reactor equipped with a condenser;
- feeding into the second reactor a stream comprising diamine so as to obtain a diacid/diamine molar ratio of between 0.9 and 1.1, the solution being brought to a temperature at most equal to the boiling point of the solution at the operating pressure by means of at least the release of heat from the reaction between the diamine and the diacid, and, optionally, feeding the amounts of additional water and/or diacid so as to obtain the salt solution at the desired concentration and with the desired diacid/diamine ratio.

2. Process according to Claim 1, **characterized in that** the concentration of dissolved species in the first reactor is between 45% and 65% by weight.

3. Process according to Claim 1 or 2, **characterized in that** the diacid/diamine molar ratio in the second reactor is between 1.00 and 1.05.

4. Process according to one of Claims 1 to 3, **characterized in that** the solution obtained in the second reactor is transferred into a third reactor preferably maintained in the absence of oxygen, equipped with a condenser, into which are added a stream comprising diamine and/or a stream comprising diacid and, optionally, a stream of water so as to adjust the diacid/diamine ratio to between 0.99 and 1.01 and to adjust the concentration by weight of salt.

5. Process according to one of Claims 1 to 4, **characterized in that** at least the second and third reactors are maintained under an oxygen-free atmosphere.

6. Process according to one of the preceding claims, **characterized in that** at least the second and third reactors are under an oxygen-free atmosphere made up of nitrogen, a rare gas, steam or a mixture thereof.

7. Process according to one of the preceding claims, **characterized in that** it operates in batch mode.

8. Process according to Claim 7, **characterized in that** the aqueous solution forming the aqueous starter solution represents at least 5% by volume of the desired amount of solution produced in the reactor at the end of the first stage.

9. Process according to Claim 8, **characterized in that** the aqueous solution forming the aqueous starter solution represents between 5% and 40% by weight, preferably between 10% and 35% by weight, of the desired amount of solution produced in the reactor at the end of the first stage.

10. Process according to one of the preceding claims, **characterized in that** the temperature in the second reactor is equal at most to the boiling point of the solution at the operating pressure, the condenser generating a reflux of condensed water.

11. Process according to one of the preceding claims, **characterized in that** the temperature in the third reactor is equal at most to the boiling point of the solution at the operating pressure, the condenser generating a reflux of condensed water.

12. Process according to one of the preceding claims, **characterized in that** the stream comprising diamine fed into the first reactor is an aqueous solution of diamine.

13. Process according to Claim 12, **characterized in that** the concentration of diamine in the abovementioned aqueous solution is at least 50%, and preferably at least 85% by weight.

14. Process according to either of Claims 1 and 6, **characterized in that** the process operates in continuous mode.

15. Process according to Claim 14, **characterized in that** the stream comprising diamine fed into the first reactor consists at least partially of the concentrated salt solution produced by the process.

16. Process according to Claim 14 or 15, **characterized in that** the second reactor comprises an external circulation loop comprising at least one static mixer.

17. Process according to Claim 16, **characterized in that** the feed of the stream comprising diamine into the second reactor is located upstream of the static mixer.

18. Process according to Claim 17, **characterized in that** the feed of the stream of salt solution originating from the first reactor is located upstream of the diamine feed.

19. Process according to one of Claims 16 to 18, **characterized in that** a device for measuring the pH of the solution is present downstream of the static mixer, and **in that** the stoichiometry is adjusted by feeding a stream of diamine and/or of diacid, downstream of the point at which the pH is measured and as a function of this measurement.

20. Process according to one of the preceding claims, **characterized in that** the diamine is hexamethylene diamine and the diacid is adipic acid.

## Patentansprüche

1. Verfahren zur Herstellung einer wässrigen Lösung von durch Mischen einer Disäure und eines Diamins erhaltenen Salzen von Diaminen und Disäuren mit einer Salzkonzentration zwischen 50 und 80 Gew.-%, **dadurch gekennzeichnet, dass** es darin besteht, dass man:
- in einem ersten Reaktor eine wässrige Lösung von Diamin und Disäure mit einem Disäure/Diamin-Molverhältnis zwischen 1,5 und 5 und einer Konzentration der gelösten Spezies in Wasser zwischen 40 und 75 Gew.-% herstellt, indem man in den Reaktor, der entweder mindestens 5 Vol.- % wässrige Lösung von Diamin und Disäure mit einem Disäure/Diamin-Molverhältnis zwischen 1,5 und 5 oder Wasser, das mindestens 10% der in den Reaktor einzuspeisenden Wassergesamtmenge entspricht, enthält, einen Strom, der Disäure umfasst, einen Strom, der Diamin umfasst, und gegebenenfalls einen Wasserstrom bei der Temperatur T₁ einspeist, wobei man die Strömungsraten des Disäure umfassenden Einspeisungsstroms und des Diamin umfassenden Einspeisungsstroms so steuert, dass die Temperatur der Lösung im Reaktor konstant unter der Siedetemperatur bei dem Betriebsdruck des Reaktors liegt und das Disäure/Diamin-Molverhältnis über 1,1 liegt, wobei die eingespeiste Säuremenge mindestens 90 Gew.-% der zur Herstellung der gewünschten Menge an wässriger Salzlösung benötigten Säuregesamtmasse entspricht und die eingespeiste Wassermenge mindestens 75 Gew.-% der zur Herstellung der gewünschten Menge an wässriger Salzlösung benötigten Wassergesamtmasse entspricht,
- die in dem ersten Reaktor erhaltene wässrige Lösung in einen zweiten Reaktor, der mit einem Kondensator ausgestattet ist, überführt,
- in den zweiten Reaktor einen Strom, der Diamin umfasst, zur Erreichung eines Disäure/Diamin-Molverhältnisses zwischen 0,9 und 1,1 einspeist, wobei die Lösung durch mindestens die Freisetzung von Wärme der Reaktion zwischen dem Diamin und der Disäure auf eine Temperatur von höchstens der Siedetemperatur der Lösung bei dem Betriebsdruck gebracht wird, und gegebenenfalls die Mengen an zusätzlichem Wasser und/oder zusätzlicher Säure zum Erhalt der Salzlösung mit der gewünschten Konzentration und mit dem gewünschten Disäure/Diamin-Verhältnis einspeist.

2. Verfahren nach Anspruch 1, **dadurch gekennzeichnet, dass** die Konzentration an gelösten Spezies in dem ersten Reaktor zwischen 45 und 65 Gew.-% liegt.

3. Verfahren nach Anspruch 1 oder 2, **dadurch gekennzeichnet, dass** das Disäure/Diamin-Molverhältnis in dem zweiten Reaktor zwischen 1,00 und 1,05 liegt.

4. Verfahren nach einem der Ansprüche 1 bis 3, **dadurch gekennzeichnet, dass** man die in dem zweiten Reaktor erhaltene Lösung in einen dritten Reaktor, der vorzugsweise unter Sauerstoffausschluss gehalten wird und mit einem Kondensator ausgestattet ist, überführt, wobei in den Reaktor ein Diamin umfassender Strom und/oder ein Disäure umfassender Strom und gegebenenfalls ein WasserStrom zur Einstellung des Disäure/Diamin-Molverhältnisses auf einen Wert zwischen 0,99 und 1,01 und zur Einstellung der Gewichtskonzentration an Salz eingetragen wird.

5. Verfahren nach einem der Ansprüche 1 bis 4, **dadurch gekennzeichnet, dass** man mindestens den zweiten Reaktor und den dritten Reaktor unter sauerstofffreier Atmosphäre hält.

6. Verfahren nach einem der vorhergehenden Ansprüche, **dadurch gekennzeichnet, dass** mindestens der zweite Reaktor und der dritte Reaktor unter einer sauerstofffreien Atmosphäre, die aus Stickstoff, einem Edelgas, Wasserdampf oder einer Mischung davon besteht, stehen.

7. Verfahren nach einem der vorhergehenden Ansprüche, **dadurch gekennzeichnet, dass** es diskontinuierlich betrieben wird.

8. Verfahren nach Anspruch 7, **dadurch gekennzeichnet, dass** die die wässrige Vorlagelösung bildende wässrige Lösung mindestens 5 Vol.-% der gewünschten Lösungsmenge, die in dem Reaktor am Ende des ersten Schritts produziert wird, entspricht.

9. Verfahren nach Anspruch 8, **dadurch gekennzeichnet, dass** die die wässrige Vorlagelösung bildende wässrige Lösung zwischen 5 und 40 Gew.-% und vorzugsweise zwischen 10 und 35 Gew.-% der gewünschten Lösungsmenge, die in dem Reaktor am Ende des ersten Schritts produziert wird, entspricht.

10. Verfahren nach einem der vorhergehenden Ansprüche, **dadurch gekennzeichnet, dass** die Temperatur in dem zweiten Reaktor höchstens gleich der Siedetemperatur der Lösung bei dem Betriebsdruck ist, wobei der Kondensator einen Rücklauf von kondensiertem Wasser erzeugt.

11. Verfahren nach einem der vorhergehenden Ansprüche, **dadurch gekennzeichnet, dass** die Temperatur in dem dritten Reaktor höchstens gleich der Siedetemperatur der Lösung bei dem Betriebsdruck ist, wobei der Kondensator einen Rücklauf von kondensiertem Wasser erzeugt.

12. Verfahren nach einem der vorhergehenden Ansprüche, **dadurch gekennzeichnet, dass** es sich bei dem in den ersten Reaktor eingespeisten Strom, der Diamin umfasst, um eine wässrige Lösung von Diamin handelt.

13. Verfahren nach Anspruch 12, **dadurch gekennzeichnet, dass** die Diaminkonzentration in der oben erwähnten wässrigen Lösung mindestens 50 Gew.-% und vorzugsweise mindestens 85 Gew.-% beträgt.

14. Verfahren nach einem der Ansprüche 1 bis 6, **dadurch gekennzeichnet, dass** es kontinuierlich betrieben wird.

15. Verfahren nach Anspruch 14, **dadurch gekennzeichnet, dass** der in den ersten Reaktor eingespeiste Strom, der Diamin umfasst, zumindest teilweise aus der durch das Verfahren produzierten konzentrierten Salzlösung besteht.

16. Verfahren nach Anspruch 14 oder 15, **dadurch gekennzeichnet, dass** der zweite Reaktor eine externe Umwälzschleife mit mindestens einem statischen Mischer aufweist.

17. Verfahren nach Anspruch 16, **dadurch gekennzeichnet, dass** sich die Einspeisung des Stroms, der Diamin umfasst, in den zweiten Reaktor stromaufwärts des statischen Mischers befindet.

18. Verfahren nach Anspruch 17, **dadurch gekennzeichnet, dass** sich die Einspeisung des Stroms von Salzlösung aus dem ersten Reaktor stromaufwärts der Diamineinspeisung befindet.

19. Verfahren nach einem der Ansprüche 16 bis 18, **dadurch gekennzeichnet, dass** stromabwärts des statischen Mischers eine Einrichtung zur Messung des pH-Werts der Lösung vorhanden ist und die Stöchiometrie durch Einspeisung eines Stroms von Diamin und/oder Disäure stromabwärts des pH-Messpunkts und in Abhängigkeit von dieser Messung eingestellt wird.

20. Verfahren nach einem der vorhergehenden Ansprüche, **dadurch gekennzeichnet, dass** es sich bei dem Diamin um Hexamethylendiamin handelt und es sich bei der Disäure um Adipinsäure handelt.
